(19) Europäisches Patentamt
European Patent Office
Office européen des brevets

(11) Publication number: **0 375 045 A1**

# (12) EUROPEAN PATENT APPLICATION

(21) Application number: **89203204.6**

(22) Date of filing: **14.12.89**

(51) Int. Cl.5: **C07D 471/06, C07D 513/06, A61K 31/435, A61K 31/54, A61K 31/55, C07D 487/06, //(C07D471/06,221:00,209:00), (C07D487/06,223:00,209:00), (C07D513/06,279:00,209:00)**

(30) Priority: **22.12.88 NL 8803136**
**18.04.89 NL 8900964**

(43) Date of publication of application:
**27.06.90 Bulletin 90/26**

(84) Designated Contracting States:
**AT BE CH DE ES FR GB GR IT LI LU NL SE**

(71) Applicant: **DUPHAR INTERNATIONAL RESEARCH B.V**
**C.J. van Houtenlaan 36**
**NL-1381 CP Weesp(NL)**

(72) Inventor: **Hamminga, Derk**
**c/o Octrooibureau Zoan B.V. P.O. Box 140**
**NL-1380 AC Weesp(NL)**
Inventor: **Van Wijngaarden, Ineke**
**c/o Octrooibureau Zoan B.V. P.O. Box 140**
**NL-1380 AC Weesp(NL)**
Inventor: **Haeck, Hans Heinz**
**c/o Octrooibureau Zoan B.V. P.O. Box 140**
**NL-1380 AC Weesp(NL)**
Inventor: **Wouters, Wouter**
**c/o Octrooibureau Zoan B.V. P.O. Box 140**
**NL-1380 AC Weesp(NL)**

(74) Representative: **Muis, Maarten et al**
**OCTROOIBUREAU ZOAN B.V. P.O. Box 140**
**NL-1380 AC Weesp(NL)**

## (54) New annelated indole derivatives.

(57) The invention relates to a group of new annelated indole derivatives of the formula

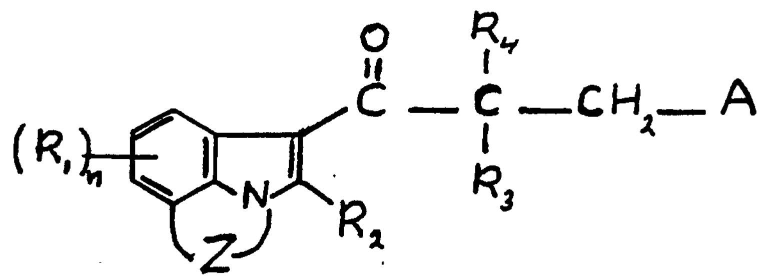

having an antagonistic activity on 5-HT receptors. The compounds can be used for the treatment of symptoms which are caused by excessive stimulation of said receptors in the gastrointestinal system, the central nervous system, the cardiovascular system, the respiratory system, and for alleviating or preventing withdrawal symptoms which are induced by abuse of drugs.

EP 0 375 045 A1

## New annelated indole derivatives

The invention relates to a group of new annelated indole derivatives which comprise an imidazolyl methyl group as a substituent, to the preparation thereof, and to compositions which comprise at least one of these compounds that compounds of formula 1

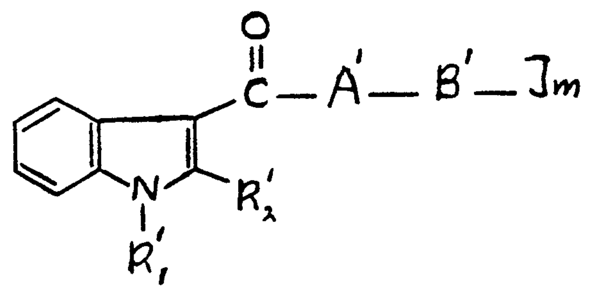

(1)

wherein $R_1'$ is hydrogen, alkyl(1-6C), alkenyl or alkynyl (3-6C), cycloalkyl(3-7C), cycloalkyl(3-7C)alkyl(1-4C), phenyl or phenylalkyl(1-3C), $R_2'$ is hydrogen, alkyl (1-6C), alkenyl(3-6C), cycloalkyl(3-7C), phenyl or phenylalkyl(1-3C), $-A'-B'$ is the group $-R_3'R_4'C-CH_2$-or $R_3'C=CH-$, wherein $R_3'$ and $R_4'$ are hydrogen or alkyl (1-6C), and Im is an optionally substituted imidazole radical, are strong antagonists of the activity of 5-hydroxytryptamine(5-HT) of "neuronal" 5-HT receptors.

It has now been found surprisingly that compounds of formula 2

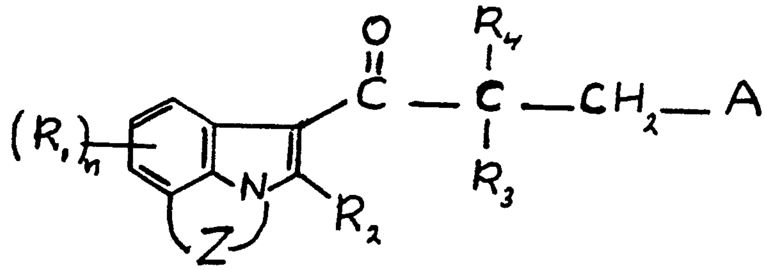

(2)

wherein

- $R_1$ is alkyl or alkoxy having 1-4 C-atoms, hydroxy, halogen, trifluoromethyl, a group $R_5S(O)_m$, wherein $R_5$ is alkyl having 1-4 C-atoms and m has the value 0, 1 or 2, a group $R_6R_7N$ or $R_6R_7$-N-CO; wherein $R_6$ and $R_7$ are hydrogen or alkyl having 1-4 C-atoms or wherein $R_6R_7N$ is a saturated 5-6 ring, and n has the value 0, 1 or 2;
- $R_2$ represents a hydrogen atom or (1-6C)alkyl, (3-6C)alkenyl or (3-7C)cycloalkyl;
- $R_3$ is a hydrogen atom or an alkyl group having 1-4 C atoms, or
- $R_2$ + $R_3$ together represent a group $-(CH_2)p-$ wherein p has the value 1,2,3 or 4;
- $R_4$ is hydrogen or alkyl having 1-4 C-atoms;
- Z, together with the carbon atom and the nitrogen atom and the intermediate carbon atom, forms a heterocyclic group consisting of 5-8 ring atoms wherein, in addition to the nitrogen atom already present, a second herero atom from the group N, O, S, S-O or $SO_2$ may be present, which ring may be substituted with 1-4 alkyl groups having 1-4 C-atoms, a phenyl group or a spiroalkyl group (2-5C), or which ring may be annelated with a saturated or non-saturated carbocyclic or heterocyclic ring which consists of 5- or 6-ring atoms and which may be substituted;
- A is a group of the formula 3, 4 or 5

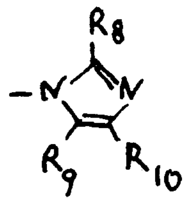
(3)

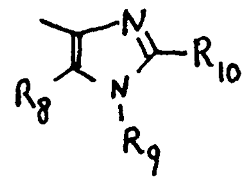
(4)

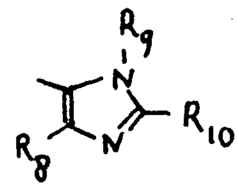
(5)

wherein $R_8$, $R_9$ and $R_{10}$ independently of each other are hydrogen, alkyl having 1-6 C-atoms, on the understanding that A is a group of the formula 4 or 5 when $R_2$ + $R_3$ together represent a group $-(CH_2)_p-$, and the pharmaceutically acceptable acid addition salts thereof have a similar but considerably prolonged activity and a lower toxicity than the known compounds of formula 1.

Examples of suitable acids with which the compounds of formula 2 according to the invention can form pharmaceutically acceptable acid addition salts are hydrochloric acid, sulphuric acid, phosphoric acid, nitric acid, and organic acids, for example, citric acid, fumaric acid, maleic acid, tartaric acid, acetic acid, benzoic acid, p-toluene sulphonic acid, methane sulphonic acid, and the like.

In the case in which the carbon atom to which the imidazolyl methyl group is bound is a chiral centre and/or when the ring Z is substituted and/or annelated, one or more chiral centres may be present. The present invention includes both the racemates and the individual enantiomers of compounds of formula 2.

The antagonistic activity of the compounds of formula 2 on the 5-HT-induced response was determined and measured in the von Bezold-Jarisch reflex-test in rats. The affinity to "neuronal" 5-HT receptors was determined and measured by the displacement of ($^3$H)GR 38032 F of neuroblastoma cells.

On the basis of the antagonistic activity on this type of 5-HT receptors, the compounds may be used for the treatment of symptoms which are caused by excessive stimulation of the said receptors a) in the gastrointestinal system (nausea and vomitting as a result of exogenous factors, for example, cancer therapy, or endogenous factors, for example, stasis of the stomach and migraine), ulcer, dyspepsia, spasms, irritable bowel syndrome, etc., or b) in the central nervous system (hallucinations, delusions, depressions, manias, fear, pain, improvement of the vigilance or the mood, etc.), or c) in the cardiovascular system, for example, spasms of the vessels, arrhythmias, etc. , or d) in the respiratory system (including nasal disturbances and disturbances of bronchi and lungs, or e) for alleviating or preventing withdrawal symptoms which are induced by abuse of drugs.

The compounds according to the invention and their salts can be brought into a form suitable for administration, for example, pills, tablets, coated tablets, capsules, powders, injection liquids, and the like, by means of techniques conventionally used for this purpose and while using suitable auxiliary substances, for example, solid or liquid carrier materials.

The dosages in which the compounds according to the invention may be used depend on the severity and the nature of the disease to be treated and on the mode of admininstration. As a rule the dosage will be between 0.05 and 20 mg, preferably between 0.1 and 10 mg, of active substance daily.

The compounds according to the invention may be prepared in a manner known for analogous compounds. Suitable methods for the preparation of this type of compounds are described, for example, in European Patent Applications no. 0266899 and 0276163.

In particular, compounds of formula 2, wherein A is a group of formula 3, can be obtained in a good yield by reaction of a compound of formula 6

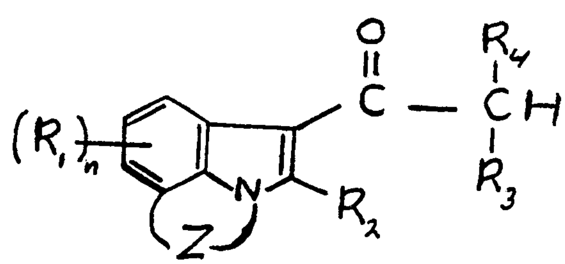
(6)

wherein $R_1$, $R_2$, $R_3$, $R_4$, n and Z have the meanings mentioned hereinbefore, on the understanding that $R_2$

+ $R_3$ together do not form a cyclic group, with dimethylamine hydrochloride and formalin, preferably in a suitable solvent, for example glacial acetic acid or ethanolic hydrochloric acid, at temperatures between 20 and 150° C, followed by reaction of the obtained Mannich base with a compound having formula 7

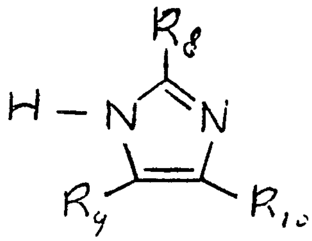

(7)

wherein $R_8$, $R_9$ and $R_{10}$ have the meanings mentioned hereinbefore, preferably in a suitable solvent, for example, water, diluted alcohol, dimethylformamide, etc., at temperatures between 20° C and 150° C.

The starting substances of formula 6 are known compounds or can be obtained in a manner known per se, for example,

1) by reaction of compounds of formula 8

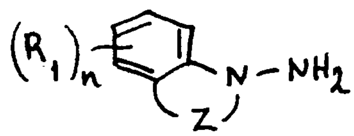

(8)

or a salt thereof, wherein $R_1$, n and Z have the meanings mentioned hereinbefore,
with a compound of formula 9

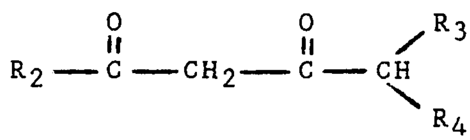

(9)

wherein $R_2$, $R_3$ and $R_4$ have the meanings mentioned hereinbefore.

This reaction may be carried out by boiling in an organic solvent, for example, acetic acid or alcohol, whether or not in the presence of an acid catalyst, for example, hydrochloric acid or sulphuric acid.

The compounds of formula 8 are known compounds or may be prepared in a manner analogous to known compounds.

2) by reaction of an acylating derivative of an acid of formula 10

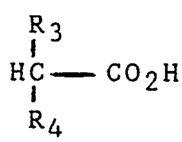

(10)

wherein $R_3$ and $R_4$ have the meanings mentioned hereinbefore, with a compound of formula 11

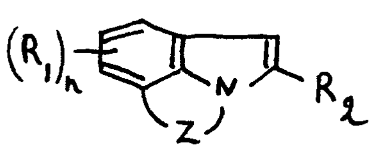

(11)

wherein $R_1$, $R_2$, n and Z have the meanings mentioned hereinbefore.

Suitable acylating derivatives of an acid of formula 10 are, for example, acid chlorides, or tertiary acid

amides in the presence of phosphorus trichloride or phosphorus oxychloride.

The reaction may be carried out in a suitable solvent, for example, pyridine, in the suitable tertiary amide of the acid of formula 10, or in dichloroethane etc. at temperatures between 0 and 120° C.

The starting compounds of formula 11 are known compounds or may be obtained in a manner analogous to known compounds, for example, by reaction of compounds of formula 8 with a compound of formula 12

$$R_2-\overset{O}{\overset{\|}{C}}-CH_3 \quad (12)$$

wherein $R_1$, $R_2$, n and Z have the meanings mentioned hereinbefore.

The reaction may be carried out by boiling in an organic solvent, for example, acetic acid or alcohol in the presence of an acid catalyst, for example, hydrochloric acid or sulphuric acid.

The compounds of formula 2, wherein A is a group of formula 4 or 5, may be prepared in a manner known for analogous compounds. Suitable modes of preparation are described, for example, in the European Patent Application no. 0242973.

In particular, these compounds can be prepared in a good yield

A) when $R_4$ is hydrogen and $R_1$, $R_2$, $R_3$, n and Z have the meanings given in formula 2, and A is a group of the formula 4 or 5, i) by metalating a compound having formula 6, wherein $R_4$ is hydrogen, and subsequent reaction of the obtained organometallic compound with a compound of the formula 13 or 14

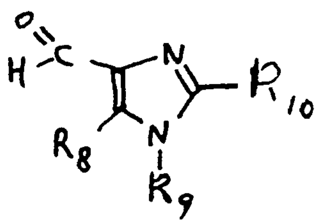

(13)

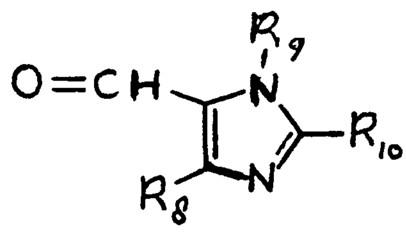

(14)

wherein $R_8$, $R_9$ and $R_{10}$ have the meanings given in formulae 4 and 5, or wherein $R_9$ is the triphenylmethyl group, on the understanding that $R_9$ cannot be hydrogen, and converting the reaction product in acid conditions, for example with methane sulphonic acid in a solvent, into a compound having formula 15 or 16

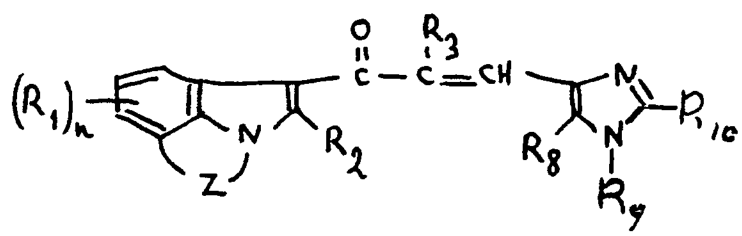

(15)

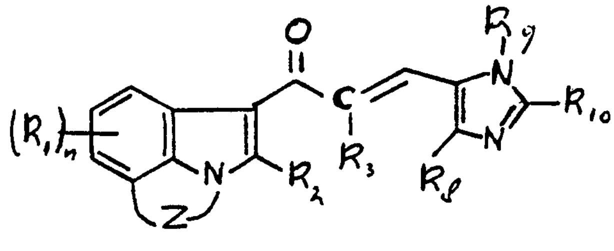

(16)

removing an optionally present triphenylmethyl group, and hydrogenating the obtained product, for example with palladium on carbon as a catalyst, preferably in a solvent, for example methanol, or

ii) by reacting a compound having formula 6, wherein $R_4$ is hydrogen, with a compound having formula 13 or 14 wherein $R_8$-$R_{10}$ have the meanings given in i) above, preferably in a solvent, for example ethanol, in the presence of a base, for example sodium hydroxide, followed by removing an optionally present triphenylmethyl group from the obtained compound having formula 15 or 16 in acid conditions, and hydrogenating, for example with palladium on carbon as a catalyst, preferably in a solvent, for example methanol.

B) When $R_1$, $R_2$, $R_3$, n and Z have the meanings given in formula 2, on the understanding that $R_2$ + $R_3$ are

not a cyclic group, and $R_4$ is hydrogen compounds having formula 2 can be obtained by reacting an acylating derivative of an acid having formula 17

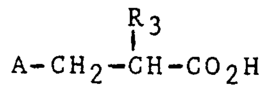

(17)

wherein $R_3$ is hydrogen or alkyl having 1-4 C-atoms, and A is a group of the formula 4 or 5 wherein $R_8$-$R_{10}$ have the meanings given in formulae 4 and 5, with a compound of the formula 11, wherein $R_1$, $R_2$, n and Z have the meaning given in formula 2.

Suitable acylating derivatives of an acid having formula 17 are for example acid chlorides or tertiary acid amides, in the presence of phosphorus trichloride or phosphorus oxychloride.

The reaction may be carried out in a suitable solvent, for example pyridine or dichloro ethane, etc., at temperatures between 20 and 120° C.

C) When $R_1$-$R_4$, $R_8$, $R_{10}$, n and Z have the meanings given in formula 2, and $R_9$ is an alkyl group, compounds having formula 2 can be obtained by metalation of the corresponding compound wherein $R_9$ is a hydrogen atom, and subsequent reaction of the resulting metal compound with a compound of formula $R_9$ - X wherein $R_9$ has the meaning mentioned in formula 2 and wherein X is a group which may be replaced by a nucleophile, for example, a halogen atom. The reaction is preferably carried out in a solvent, for example, dimethylformamide, dimethylsulphoxide, etc. with metalation reagents, for example, sodium hydride, potassium tertiary butoxide, sodium methanolate etc.

D) When $R_1$-$R_3$, $R_8$, n and Z have the meanings given in formula 2, $R_4$ is alkyl having 1-4 C-atoms, and $R_9$ and $R_{10}$ are hydrogen, these compounds having formula 2 can be obtained by reaction of a compound of formula 18

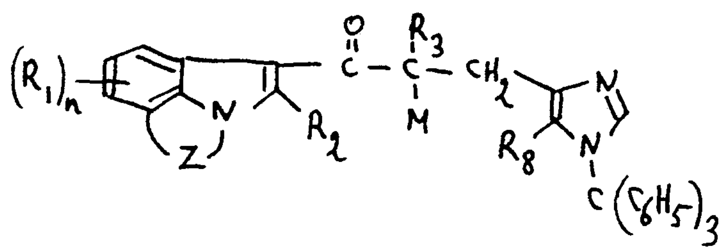

(18)

wherein $R_1$, $R_2$, $R_3$, $R_8$, n and Z have the meanings mentioned in formula 2 and M is a metal atom, with a compound of formula $R_4$-X, wherein $R_4$ is alkyl having 1-4 C and X is a group which may be replaced by a nucleophile, for example, a halogen atom, and then removing the trityl group from the resulting reaction mixture in acid conditions, for example by boiling in aqueous acetic acid.

The starting compounds of formula 18 used in this reaction may be prepared by metalation of the analogous starting compound of formula 18, wherein M is a hydrogen atom, for example, with lithium diisopropylamide or butyl lithium in a suitable solvent, for example, tetrahydrofuran.

The starting compounds of formula 18, wherein M is a hydrogen atom, can be obtained for example, by hydrogenation of the intermediates wherein $R_9$ is hydrogen of above method A ii)

The invention will now be described in greaterdetail with reference to the following examples.

## EXAMPLE I

### 1-(5,6-dihydro-4H-pyrrolo [3,2,1-ij]quinolin-1-yl)-3-(5-methyl-1H-imidazol-4-yl)propanone-1 hydrochloride

4.5 g(71.5 mmol) of ammonium formate were added to a mixture of 6 g (14.3 mmol) of N,N-dimethyl-3-[5-methyl-1-(triphenylmethyl)-1H-imidazol-4-yl]-propane amide, 3 g of 10% palladium on carbon and 125 ml of methanol, while stirring and in one portion. The reaction mixture was heated slowly and boiled for two hours. It was then cooled, filtrated over hyflo and washed with methanol and with an alcoholic hydrochloric acid solution. After evaporating the filtrate 4.5 g of semi-solid product were obtained.

The above product was suspended in 50 ml of 1,2-dichloroethane. 2.0 ml of phosphorous oxychloride (21.5 mmol) were added and the mixture was stirred for 10 minutes. 2.25 g (14.3 mmol) of 5,6-dihydro-4H-pyrrolo[3,2,1-ij]quinoline were added and the mixture was boiled for 6 hours. After cooling, water and methylene chloride were added and the whole was shaken. The water layer was separated, made alkaline with soda and shaken with methylene chloride. The methylene chloride layer was separated, dried and evaporated in vacuo.

The residue (3.7 g) was chromatographed over 200 g of silica gel using methylene chloride/ethanol/ammonia (89/10/1) as an eluent. The desired fractions were evaporated in vacuo and dissolved in a mixture of 15 ml of absolute alcolhol and 10 ml of ethyl acetate. The reaction mixture of 1 ml of acetyl chloride with 4 ml of absolute alcohol was then added. After leaving to stand overnight at 0 ° C the crystals were sucked off and washed successively with ethyl acetate and absolute alcohol, and dried.

2.5. g of the desired hydrochloride were obtained having a melting-point of 250-252 ° C (decomposition).

The following compounds were obtained in the same manner:

(1) 1-(4,5,6,7-tetrahydro-pyrrolo [3,2,1-jk]-[1]-benzazepin-1-yl)-3-(5-methyl-1H-imidazol-4-yl)-propanone-1 hydrochloride; melting point 240-241 ° C (decomposition);

(2) 1-(2,3-dihydro-pyrrolo [1,2,3-de] [1,4]benzothiazin-6-yl)-3-(5-methyl-1H-imidazol-4-yl)-propanone-1 hydrochloride; melting point 233-234 ° C;

(3) 1-(6,7-dihydro-indolo [1,7a,7-ab] [1]benzazepin-2-yl)-3-(5-methyl-1H-imidazol-4-yl)-propanone-1; C-13-NMR ($CDCl_3$, Ref: TMS)

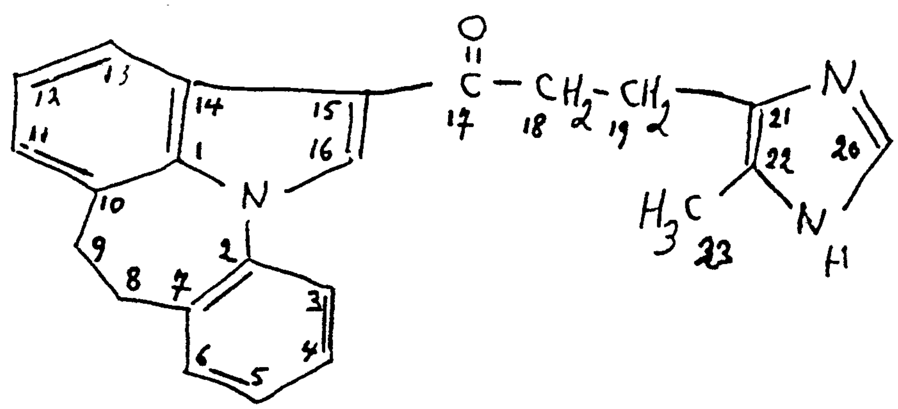

| | | | | | | | | |
|---|---|---|---|---|---|---|---|---|
| 1 | 135.45 | S | 9 | 34.03 | T | 17 | 195..71 | S |

| | | | | | | | | |
|---|---|---|---|---|---|---|---|---|
| 1 | 135.45 | S | 9 | 34.03 | T | 17 | 195.71 | S |
| 2 | 139.03 | S | 10 | 127.52 | S | 18 | 39.44 | T |
| 3 | 126.32 | D | 11 | 122.65 | D | 19 | 19.81 | T |
| 4 | 127.65 | D | 12 | 120.28 | D | 20 | 133.89 | D |
| 5 | 124.51 | D | 13 | 122.76 | D | 21 | 126.95 | S |
| 6 | 130.64 | D | 14 | 127.65 | S | 22 | 129.49 | S |
| 7 | 135.41 | S | 15 | 118.61 | S | 23 | 10.55 | Q |
| 8 | 34.66 | T | 16 | 132.49 | D | | | |

(4) 1-(4-methyl-5,6-dihydro-4H-pyrrolo[3,2,1-ij]quinolin-1-yl)-3-(5-methyl-1H-imidazol-4-yl)-propanone-1 hydrochloride; melting point 235 ° C
C-13-NMR (SLV: $CDCl_3$, Ref: TMS,

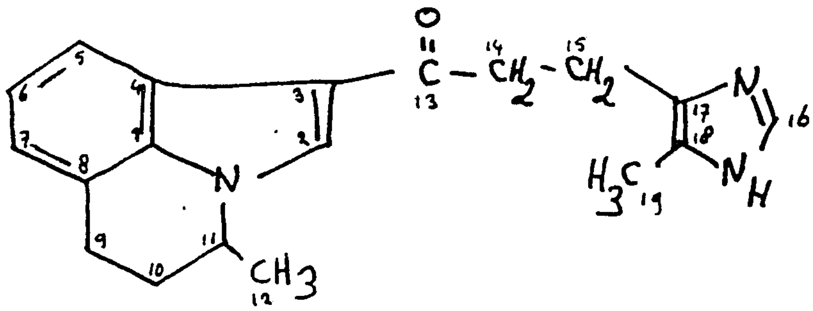

| | | | | | | | | |
|---|---|---|---|---|---|---|---|---|
| 1 | 134.73 | S | 8 | 122.23 | S | 15 | 20.28 | T |
| 2 | 131.36 | D | 9 | 23.24 | T | 16 | 132.63 | D |
| 3 | 116.84 | S | 10 | 30.48 | T | 17 | 129.88 | S |
| 4 | 124.35 | S | 11 | 50.55 | D | 18 | 127.07 | S |
| 5 | 119.59 | D | 12 | 20.32 | Q | 19 | 10.89 | Q |
| 6 | 122.79 | D | 13 | 195.57 | S | | | |
| 7 | 120.46 | D | 14 | 39.66 | T | | | |

EXAMPLE II

1-(5,6-dihydro-2-methyl-4H-pyrrolo [3,2,1-ij]quinolin-1-yl)-3-(5-methyl-1H-imidazol-4-yl)-propanone-1 hydrochloride

4.3 g (20 mmol) of 1-acetyl-2-methyl-5,6-dihydro-4H-pyrrolo {3,2,1-ij]quinoline and 10.5 g (30 mmol) of 5-methyl-1-triphenylmethyl)-1H-imidazole-4-carboxaldehyde were dissolved in 175 ml of absolute alcohol while stirring and heating. A solutiŏn of 11.5 g (0.21 mmol) of potassium hydroxide in 85 ml of water was added and the mixture was stirred for 60 hours at 50° C. The reaction mixture was diluted with 500 ml of water and shaken with methylene chloride. The methylene chloride layer was evaporated in vacuo and the residue was boiled with 250 ml of ethyl acetate and kept at room temperature for 20 hours. The solid was sucked off and dried. 9.0 g of 1-(5,6-dihydro-2-methyl-4H-pyrrolo [3,2,1-ij]quinolin-1-yl)-3-(5-methyl-1-triphenylmethyl-1H-imidazol-4-yl)-2-propen-1-one were obtained.

2.5 g (4.6 mmol) of the above product together with 1.0 g of Pd/C(10%) in a mixture of 70 ml of methanol and 20 ml of dioxan were stirred at 40° C. 1.5 g (23 mmol) of ammonium formate were added and the mixture was boiled while stirring for 30 minutes. It was then cooled, filtrated over hyflo and washed with methylene chloride and methanol which contains 3% of triethylamine. The filtrate was evaporated in vacuo and the residue was chromatographed over 95 g of silica gel using methylene chloride/ethanol/amonia (89/10/1) as an eluent. After evaporating the desired fractions, 0.67 g of a yellow oil were obtained. This oil was dissolved in 10 ml of absolute ethanol and diluted with 0.5 ml of concentrated hydrochloric acid. After a few hours the solid was sucked off, washed with absolute ethanol and dried. 0.63 g Of the desired product were obtained having a melting-point of 275° C (decomposition).
C-13-NMR ($CDCl_3$, Ref.: TMS, ADT: TEA)

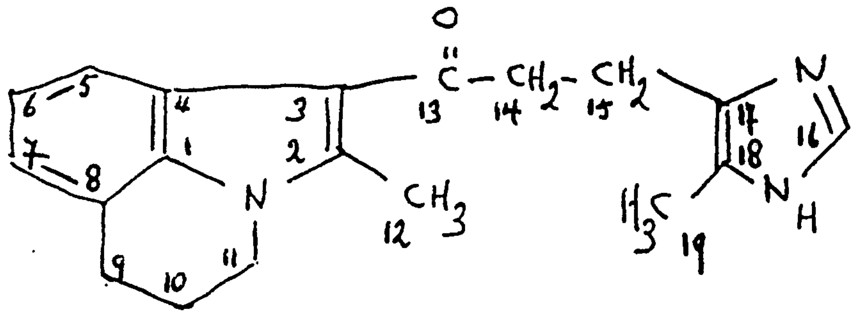

| | | | | | | | | | | | |
|---|---|---|---|---|---|---|---|---|---|---|---|
| 1 | 133.66 | S | | 8 | 122.04 | S | | 15 | 19.26 | T | |
| 2 | 144.24 | S | | 9 | 24.68 | T | | 16 | 132.54 | D | |
| 3 | 113.40 | S | | 10 | 22.50 | T | | 17 | 128.18 | S | |
| 4 | 124.45 | S | | 11 | 42.82 | T | | 18 | 128.98 | S | |
| 5 | 118.38 | D | * | 12 | 11.28 | Q | + | 19 | 12.53 | Q | + |
| 6 | 122.15 | D | * | 12 | 196.76 | S | | | | | |
| 7 | 119.51 | D | * | 14 | 41.82 | T | | | | | |

C-ATOM 16 IS SPLITTED UP <2.1 HZ>

In the same manner was obtained :

1-(2-methyl-4,5,6,7 -tetrahydro-pyrrolo[3,2,1-jk]-1-benzazepin-1-yl)-3-(5-methyl-1H-imidazol-4-yl)-propanon-1 hydrochloride; melting point 241-241.5 °C

C-13-NMR (SLV: $CDCl_3$, Ref.: TMS, ADT. Triethylamine)

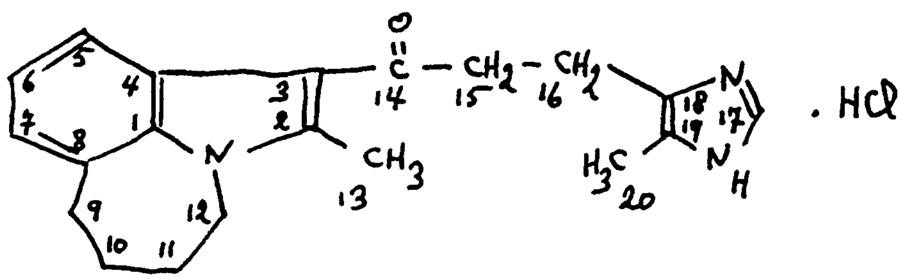

| | | | | | | | | |
|---|---|---|---|---|---|---|---|---|
| 1 | 136.62 | S | 8 | 126.48 | S | 15 | 42.76 | T |
| 2 | 144.98 | S | 9 | 27.42 | T | 16 | 19.02 | T |
| 3 | 113.75 | S | 10 | 26.00 | T | 17 | 131.81 | D |
| 4 | 127.50 | S | 11 | 30.68 | T | 18 | 127.21 | S |
| 5 | 118.61 | D | 12 | 43.21 | T | 19 | 128.89 | S |
| 6 | 122.64 | D | 13 | 12.90 | Q | 20 | 10.71 | Q |
| 7 | 122.02 | D | 14 | 196.24 | S | | | |

## EXAMPLE III

10-[(5-methyl-1H-imidazol-4-yl)methyl]-5,6,8,9,10,11-hexahydro-4H-pyrido [3,2,1-jk]carbazol-11-one.

a) 8.9 ml (14.2 mmol) of butyllithium in hexane were added dropwise to a solution of 2.0 g (14.2 mmol) of 2,2,6,6-tetramethyl-piperidine in 30 ml of dry tetrahydrofuran at -78 °C. The mixture was stirred for 30 minutes at -10 °C, and cooled once more to -78 °C. This solution was added dropwise to a solution of 2.9 g (12.9 mmol) of 5, 6,8,9,10,11-hexahydro-4H-pyrido [3,2,1-jk]carbazol-11-one in 100 ml of 8dry tetrahydrofuran at -70 °C. The mixture was stirred for 390 minutes at -70 °C. A solution of 5.0 g (14.5 mmol) of 5-methyl-1-triphenylmethyl-1H-imidazol-4-carboxaldehyde in 30 ml of dry tetrahydrofuran was added at the same temperature. Without further cooling the temperature raised to -10 °C. At this temperature the mixture was stirred for 1 hour. The reaction mixture was cooled to -70 °C and 35 ml of acetic acid (at -70 °C) and 16.9 g of p-toluenesulphonic acid (at -10 °C) were added successively, and the mixture was boiled for 20 hours. The mixture was then evaporated under reduced pressure. The residue was shaken with excess of 2N sodium hydroxide and with methylene chloride. The organic layer was separated, washed with brine, dried and evaporated to dryness. The residue was chromatographed over silica gel using methylene chloride/methanol/ammonia (92/7/1) as an eluent. After evaporating the desired fractions 1.3 g of 10-(5-methyl-1H-imidazol-4-yl)methylene-5,6,8,9,10,11-hexahydro-4H-pyrido [3,2,1-jk] carbazol-11-one were obtained.

b) This product (1.3 g; 4.1 mmol) were dissolved in a mixture of 75 ml of dimethylformamide and 100 ml of absolute ethanol, and hydrogenated with palladium on 10 % of carbon at 20 °C and 1 atm. After two hours thé mixture was filtrated over hyflo and the filtrate was evaporated in vacuo.

The residue was chromatographed over silical gel using methylene chloride/methanol/ammonia (92/7/1) as an eluent. After evaporating the desired fractions 1.05 g of the desired product was obtained; melting point 156.5-158 °C.

C-13-NMR (SLV: DMSO, Ref.: TMS)

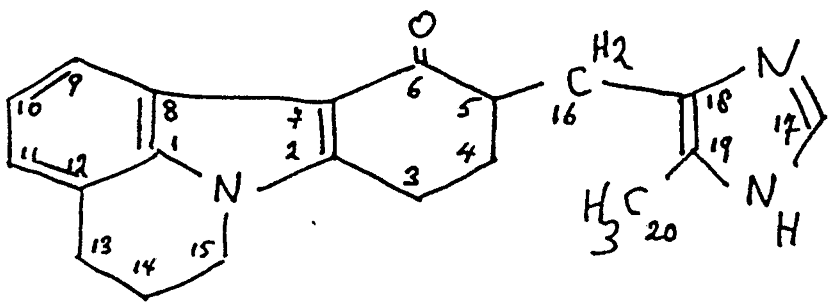

| | | | | | | | | |
|---|---|---|---|---|---|---|---|---|
| 1 | 134.12 | S | 8 | 122.13 | S | 15 | 41.34 | T |
| 2 | 150.69 | S | 9 | 121.89 | D | 16 | 25.17 | T |
| 3 | 19.75 | T | 10 | 117.66 | D | 17 | 132.76 | D |
| 4 | 27.32 | T | 11 | 119.76 | D | 18 | 126.10 | S |
| 5 | 46.65 | D | 12 | 122.49 | S | 19 | 129.00 | S |
| 6 | 193.87 | S | 13 | 23.80 | T | 20 | 10.53 | Q |
| 7 | 110.80 | S | 14 | 21.95 | T | | | |

BROAD LINES FOR CARBON 18 AND 19

In the same manner were obtained:

1) 2-fluoro-10-{(5-methyl-1H-imidazol-4-yl)methyl}-5,6,8,9,10,11-hexahydro-4H-pyrido[3,2,1-jk]carbazol-11-one; melting point 239 °C (decompostion).

C-13-NMR (SLV: $CDCl_3$, Ref. : TMS, ADT: MeOH)

C-13-NMR (SLV: $CDCl_3$, Ref.: TMS, ADT: MeOH)

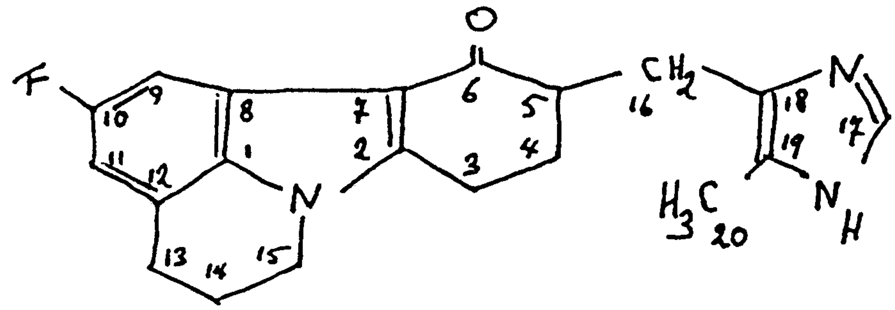

| | | | | | | | | |
|---|---|---|---|---|---|---|---|---|
| 1 | 131.10 | S | 8 | 123.19 | S | 15 | 41.93 | T |
| 2 | 151.56 | S | 9 | 104.30 | D | 16 | 24.99 | T |
| 3 | 21.05 | T | 10 | 160.06 | S | 17 | 133.00 | D |
| 4 | 28.52 | T | 11 | 108.90 | D | 18 | 126.37 | S |
| 5 | 47.05 | D | 12 | 123.06 | S | 19 | 130.22 | S |
| 6 | 195.92 | S | 13 | 24.42 | T | 20 | 11.36 | Q |
| 7 | 112.29 | S | 14 | 22.38 | T | | | |

COUPLING CONSTANTS:

J(10,F21) = 237.6
J(11, F21) = 26.2
J(9,F21) = 25.4
J( 8,F21) = 11.6
J(12, F21) = 10.2
J(7,F21) = 4.4
LINES OF C-ATOMS 18 AND 19 ARE BROAD

2) (-) 6-methyl-10-{(5-methyl-1H-imidazol-4-yl)methyl}-5,6,8,9,10,11-hexahydro-4H-pyrido[3,2,1-jk]carbazol-11-one hydrochloride; melting point 316-318 °C (decomposition)

C-13-NMR ($CDCl_3$, Ref.: TMS, ADT: TEA)

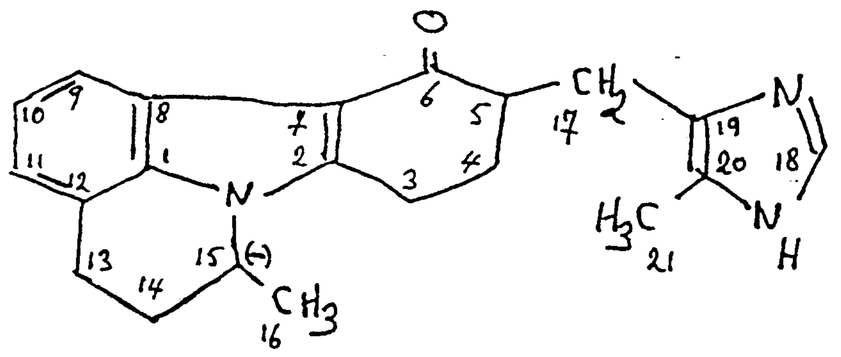

| | | | | | | | | | | | |
|---|---|---|---|---|---|---|---|---|---|---|---|
| 1 | 150.03 | S | | 8 | 112.39 | S | # | 15 | 47.29 | D | |
| 2 | 133.73 | S | | 9 | 122.52 | D | | 16 | 20.22 | Q | * |
| 3 | 20.19 | T | * | 10 | 118.60 | D | | 17 | 25.33 | T | |
| 4 | 28.19 | T | | 11 | 120.56 | D | | 18 | 132.78 | D | |
| 5 | 47.13 | D | | 12 | 123.21 | S | | 19 | 127.61 | S | |
| 6 | 195.63 | S | | 13 | 20.87 | T | | 20 | 128.92 | S | |
| 7 | 121.33 | S | # | 14 | 28.59 | T | | 21 | 11.23 | Q | |

| | | | | | | | | | | | |
|---|---|---|---|---|---|---|---|---|---|---|---|
| 1 | 150.09 | S | | 8 | 112.44 | S | # | 15 | 47.23 | D | |
| 2 | 133.86 | S | | 9 | 122.52 | D | | 16 | 20.31 | Q | * |
| 3 | 20.26 | T | * | 10 | 118.60 | D | | 17 | 25.22 | T | |
| 4 | 28.22 | T | | 11 | 120.49 | D | | 18 | 132.75 | D | |
| 5 | 47.13 | D | | 12 | 123.11 | S | | 19 | 127.52 | S | |
| 6 | 195.58 | S | | 13 | 21.23 | T | | 20 | 129.02 | S | |
| 7 | 121.42 | S | # | 14 | 28.86 | T | | 21 | 11.26 | Q | |

MIXTURE OF 2 DIASTEREOMERS

3) (+) 6-methyl-10-{(5-methyl-1H-imidazol-4-yl)methyl}-5,6,8,9,10,11-hexahydro-4H-pyrido[3,2,1-jk]-carbazol-11-one hydrochloride; melting point 317-319 °C (decomposition)
C-13-NMR (SLV: $CDCl_3$, Ref.: TMS, ADT: TEA)

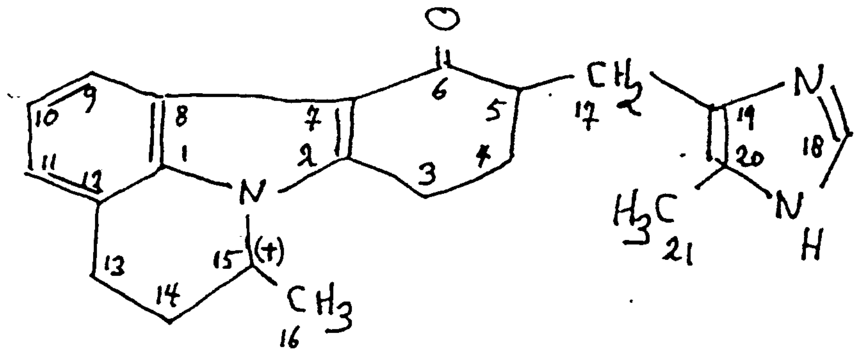

| | | | | | | | | | | |
|---|---|---|---|---|---|---|---|---|---|---|
| 1 | 150.00 | S | | 8 | 112.55 | S | # | 15 | 47.36 | D |
| 2 | 133.75 | S | | 9 | 122.67 | D | | 16 | 20.24 | Q |
| 3 | 20.19 | T | | 10 | 118.70 | D | | 17 | 25.08 | T |
| 4 | 28.23 | T | | 11 | 120:67 | D | | 18 | 132.51 | D |
| 5 | 47.10 | D | | 12 | 123.21 | S | | 19 | 126.55 | S |
| 6 | 195.71 | S | | 13 | 21.12 | T | | 20 | 129.83 | S |
| 7 | 121.31 | S | # | 14 | 28.96 | T | | 21 | 11.38 | Q |

| | | | | | | | | | | |
|---|---|---|---|---|---|---|---|---|---|---|
| 1 | 150.08 | S | | 8 | 122.58 | S | # | 15 | 47.29 | D |
| 2 | 133.90 | S | | 9 | 122.67 | D | | 16 | 20.30 | Q |
| 3 | 20.30 | T | | 10 | 118.70 | D | | 17 | 25.00 | T |
| 4 | 28.26 | T | | 11 | 120.59 | D | | 18 | 132.46 | D |
| 5 | 47.10 | D | | 12 | 123.09 | S | | 19 | 126.47 | S |
| 6 | 195.71 | S | | 13 | 21.41 | T | | 20 | 129.91 | S |
| 7 | 121.41 | S | # | 14 | 29.18 | T | | 21 | 11.38 | Q |

MIXTURE OF TWO DIASTEREOMERS

4) 11-{(5-methyl-1H-imidazol-4-yl)methyl}-5,6,9,10, 11,12-hexahydro-4H,8H-cyclohepta[4,5]pyrrolo[3,2,1-ij] quinolin-12-one
C-13-NMR (SLV: $CDCl_3$, Ref.: TMS, ADT: MeOH)

C-13-NMR (SLV: $CDCl_3$, Ref.: TMS, ADT: MeOH)

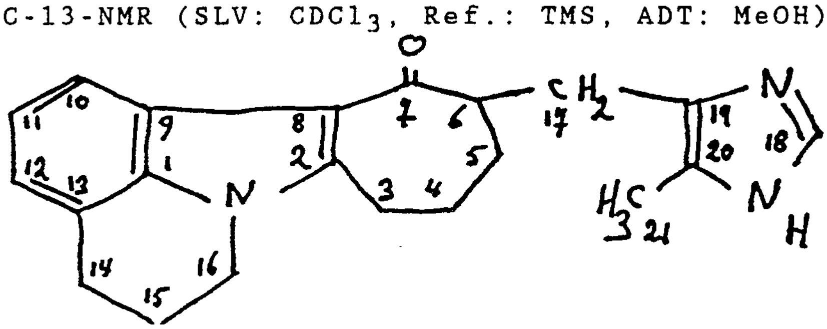

| | | | | | | | | |
|---|---|---|---|---|---|---|---|---|
| 1 | 133.75 | S | 8 | 115.31 | S | 15 | 22.82 | T |
| 2 | 148.28 | S | 9 | 121.57 | S | 16 | 42.24 | T |
| 3 | 26.26 | T # | 10 | 119.31 | D | 17 | 24.93 | T # |
| 4 | 24.35 | T | 11 | 122.79 | D | 18 | 132.60 | D |
| 5 | 28.41 | T # | 12 | 120.40 | D | 19 | *.00 | |
| 6 | 50.72 | D | 13 | 125.22 | S | 20 | *.00 | |
| 7 | 199.13 | S | 14 | 24.47 | T | 21 | 11.96 | Q |

C-ATOMS 19 AND 20 ARE NOT SHOWN (VERY BROAD)

5) 11-{(5-methyl-1H-imidazol-4-yl)methyl}-4,5,6,7,9,10, 11,12-octahydro-azepino[3,2,1-jk]carbazol-12-one; melting point 218-219 °C;

6) 3-{(5-methyl-1H-imidazol-4-yl)methyl}-1,2,3,4,8,9-hexahydro-[1]benzazepino[3,2,1-jk]carbazol- 4-one hydrochloride;

C-13-NMR (SLV: $CDCl_3$, Ref.: TMS, ADT: Triethylamine)

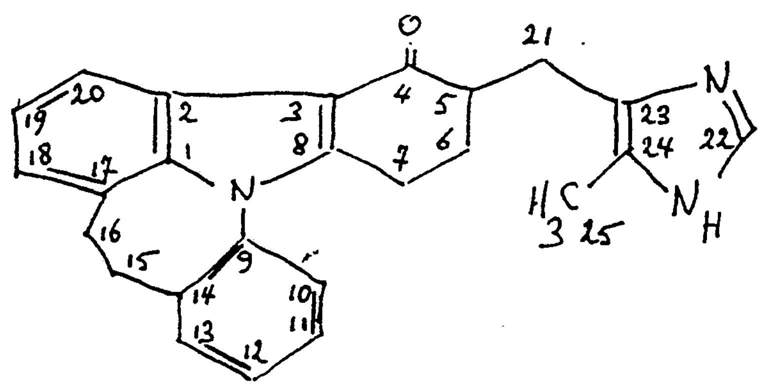

| | | | | | | | | |
|---|---|---|---|---|---|---|---|---|
| 1 | 136.96 | S | 10 | 124.95 | D # | 19 | 119.15 | D |
| 2 | 125.86 | S | 11 | 126.97 | D # | 20 | 124.56 | D # |
| 3 | 115.22 | S | 12 | 126.63 | D # | 21 | 25.11 | |
| 4 | 196.74 | S | 13 | 130.26 | D | 22 | 132.90 | D |
| 5 | 47.61 | D | 14 | 136.16 | | 23 | 127.18 | S |

| | | | | | | | | |
|---|---|---|---|---|---|---|---|---|
| 6 | 30.49 | | 15 | 34.30 | T | 24 | 129.40 | |
| 7 | 26.50 | | 16 | 34.30 | T | 25 | 11.27 | Q |
| 8 | 151.75 | S | 17 | 127.18 | S | | | |
| 9 | 137.73 | S | 18 | 122.56 | D # | | | |

MOST LINES ARE BROADENED!!

## EXAMPLE IV

1-(5,6-dihydro-2-methyl-4H-pyrrolo [3,2,1-ij]quinolin-1-yl)-3-(2-methyl-1H-imidazol-1-yl)-propanone-1

2.13 g (10 mmol) of 1-acetyl-2-methyl-5,6-dihydro-4H-pyrrolo [3,2,1-ij]quinoline together with 0.6 g (20 mmol) of paraformaldehyde and 1.8 g (22 mmol) of dimethylamine hydrochloride in 30 ml of acetic acid were stirred at 100° C for 1 hour. The mixture was then evaporated in vacuo. The residue was shaken with a mixture of methylene chloride and 2 N potassium hydroxide. The methylene chloride layer was dried and evaporated. The residue was chromatographed over silica gel using methanol which contained 3% of triethyl amine as an eluent. After evaporating the desired fractions 1.0 g of 1-(5,6-dihydro-2-methyl-4H-pyrrolo [3,2,1-ij]quinolin-1-yl)-3-(dimethylamino)-1-propanone were obtained.

The above product together with 1.0 g of 2-methyl-imidazole in a mixture of 20 ml of propanol and 20 ml of water was stirred at 100° C for 16 hours. It was then cooled, diluted with 100 ml of water and shaken with methylene chloride. The methylene chloride layer was dried and evaporated. The residue was chromatographed over 50 g of silica gel using methylene chloride which contained 5% of methanol as an eluent. After evaporating the desired fractions 0.42 g of desired product were obtained, having a melting-point of 176-178° C.

In the same manner the following compound has been prepared:
1-(2-methyl-4,5,6,7-tetrahydro-pyrrolo[3,2,1-jk]-[1]-benzazepin-1-yl)-3-(2-methyl-1H-imidazol-1-yl)-propanone-1 hydrochloride; melting point 192.5-194 ° C.

## EXAMPLE V

A solution of 1.7 g (5.8 mmol) of 1-(5,6-dihydro-4H-pyrrolo[3,2,1-ij]quinolin-1-yl)-3-(5-methyl-1H-imidazol-4-yl)-propanone-1 in 15 ml of dimethylformamide was added dropwise to a suspension of 0.24 g (10 mmol) of sodium hydride (55 % in oil) in 7.5 ml of dimethylformamide at 10-15 ° C. The mixture was stirred for 20 minutes at 20 ° C. 0.49 ml (6 mmol) of allyl chloride was added then, and the mixture was stirred for 3 hours at 20 ° C. Another 0.1 ml of allyl chloride was added, and the mixture was stirred for 20 hours, diluted with water and shaken with methylene chloride. The organic layer was evaporated in vacuo and the residue was chromatographed over silica gel using methylene chloride/ethanol/ammonia (250/10/1) as an eluent. After evaporating the desired fractions 1.2 g of the desired mixture were obtained.

This mixture was separated by means of HPLC. In this manner both isomers were obtained
a) 1-(5,6-dihydro-4H-pyrrolo[3,2,1-ij]quinolin-1-yl)-3-(1-allyl-5-methyl-1H-imidazol-4-yl)-propanone-1; (yield 0.2 g)
C-13-NHR (SLV: $CDCl_3$, Ref.: TMS

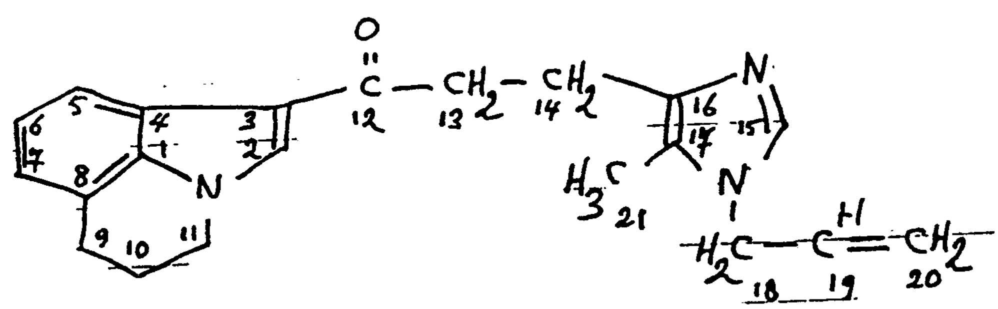

| | | | | | | | | | | | |
|---|---|---|---|---|---|---|---|---|---|---|---|
| 1 | 134.74 | S | | 8 | 121.99 | S | * | 15 | 135.16 | D | |
| 2 | 132.95 | D | | 9 | 24.39 | T | | 16 | 137.26 | S | |
| 3 | 117.02 | S | | 10 | 22.71 | T | | 17 | *.00 | | |
| 4 | 124.28 | S | * | 11 | 44.75 | T | | 18 | 47.15 | T | |
| 5 | 120.05 | D | # | 12 | 195.67 | S | | 19 | 132,98 | D | |
| 6 | 122.80 | D | # | 13 | 39.73 | T | | 20 | 117.35 | T | |
| 7 | 120.35 | D | # | 14 | 22.75 | T | | 21 | 8.15 | Q | |

CS OF CARBON 17 IS MAYBE 121.99 PPM

b) 1-(5,6-dihydro-4H-pyrrolo[3,2,1-ij]quinolin-1-yl)-3-(1-allyl-4-methyl-1H-imidazol-5-yl)-propanone-1 (yield 0.28 g)
C-13-NMR (SLV: $CDCl_3$, Ref.: TMS)

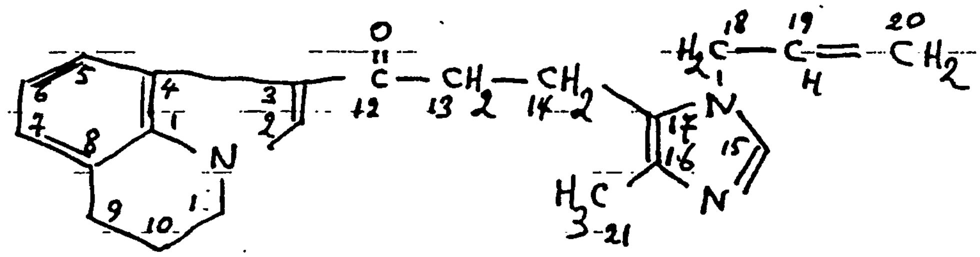

| | | | | | | | | | | | |
|---|---|---|---|---|---|---|---|---|---|---|---|
| 1 | 134.78 | S | | 8 | 122.29 | S | # | 15 | 135.25 | D | |
| 2 | 132.31 | D | | 9 | 24.35 | T | | 16 | 134.27 | S | |
| 3 | 116.72 | S | | 10 | 22.72 | T | | 17 | 126.05 | S | |
| 4 | 124.10 | S | # | 11 | 44.89 | T | | 18 | 47.19 | T | |
| 5 | 119.76 | D | * | 12 | 193.97 | S | | 19 | 133.58 | D | |
| 6 | 123.09 | D | * | 13 | 39.56 | T | | 20 | 117.53 | T | |
| 7 | 120.64 | D | * | 14 | 17.94 | T | | 21 | 13.02 | Q | |

## Claims

1. Annelated indole derivatives of formula 2

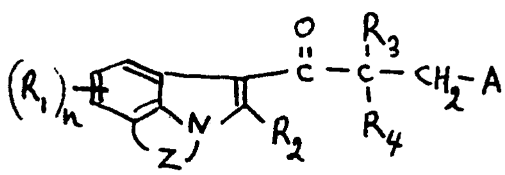

(2)

wherein

- $R_1$ is alkyl or alkoxy having 1-4 C-atoms, hydroxy, halogen, trifluoromethyl, trifluoromethoxy, trifluoromethylthio, a group $R_5S(O)_m$, wherein $R_5$ is alkyl having 1-4 C-atoms and m has the value 0,1 or 2, a group $R_6R_7N$ or $R_6R_7$-N-CO, wherein $R_6$ and $R_7$ are hydrogen or alkyl having 1-4 C-atoms or wherein $R_6R_7N$ is a saturatured 5-6 ring, and n has the value 0,1 or 2;
- $R_2$ is a hydrogen atom or a (1-6C)alkyl, a (3-6C)alkenyl or (3-7C)cycloalkyl;
- $R_3$ is a hydrogen atom or an alkyl group having 1-4 C atoms, or
- $R_2$ + $R_3$ together represent a group $-(CH_2)_p-$ wherein p has the value 1,2,3 or 4;
- $R_4$ is hydrogen or alkyl having 1-4 C-atoms;
- Z, together with the carbon atom and the nitrogen atom and the intermediate carbon atom, forms a heterocyclic group consisting of 5-8 ring atoms wherein, in addition to the nitrogen atom already present, a second herero atom from the group N, O, S, S-O or $SO_2$ may be present, which ring may be substituted with 1-4 alkyl groups having 1-4 C-atoms, a phenyl group or a spiroalkyl group (2-5C), or which ring may be annelated with a saturated or non-saturated carbocyclic or heterocyclic ring which consists of 5- or 6-ring atoms and which may be substituted;
- A is a group of the formula 3, 4 or 5

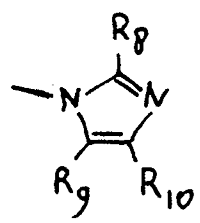

(3)

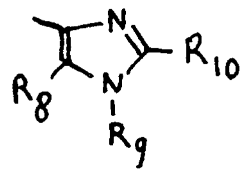

(4)

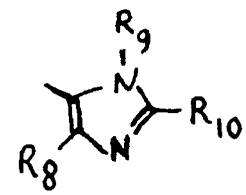

(5)

wherein $R_8$, $R_9$ and $R_{10}$ independently of each other are hydrogen, alkyl having 1-6 C-atoms, on the understanding that A is a group of the formula 4 or 5 when $R_2$ + $R_3$ together represent a group $-(CH_2)_p-$, and pharmacologically acceptable acid addition salts thereof.

2. Pharmaceutical compositions which comprise an annelated indole derivative as an active substance, characterised in that they comprise at least one compound as claimed in Claim 1 as an active substance.

3. A method of preparing pharmaceutical compositions by bringing an active substance in a form suitable for administration, characterised in that at least one compound as claimed in Claim 1 is used as an active substance.

4. A method of treating symptoms which are caused by excessive stimulation of 5-hydroxytryptamine receptors, characterised in that a compound as claimed in Claim 1 is used.

5. A method of preparing annelated indole derivatives, characterised in that compounds of formula 2 are prepared in which A is a group of formula 3, by reaction of a compound of formula 6

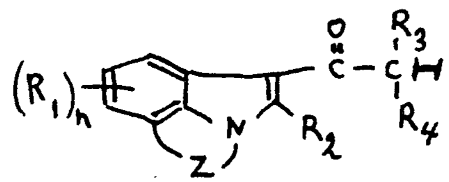

(6)

wherein $R_1$, $R_2$, $R_3$, $R_4$, n and Z have the meanings mentioned in Claim 1 on the understanding that $R_2$ + $R_3$ do not constitute a cyclic group, with dimethylamine hydrochloride and formalin followed by reacting the Mannich-base with a compound of formula 7

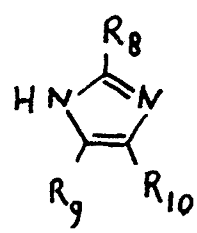

(7)

wherein $R_8$, $R_9$ and $R_{10}$ have the meanings mentioned in Claim 1.

6. A method as claimed in Claim 5, characterised in that compounds of formula 2 are prepared, wherein A is a group of formula 4 or 5

a) by reaction of a compound having formula 6 wherein $R_4$ is hyrogen or a metal atom, with a compound of the formula 13 or 14

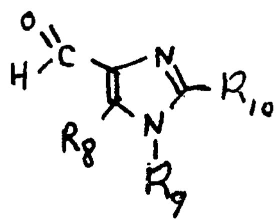

(13)

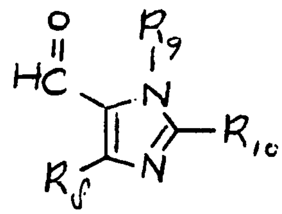

(14)

wherein $R_8$, $R_9$ and $R_{10}$ have the meaning given in claim 1, or $R_9$ is the triphenylmethyl group, on the understanding that $R_9$ is no hydrogen atom, and converting the reaction product under acid conditions into a compound of the formula 15 or 16

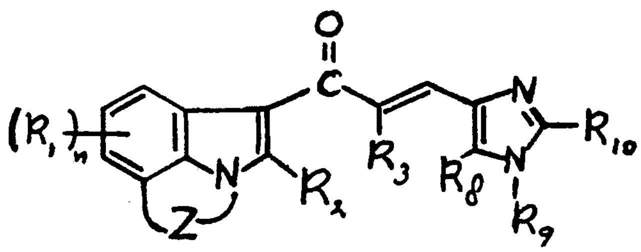

(15)

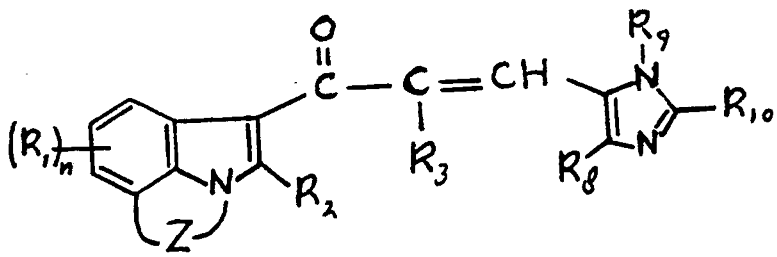

(16)

and hydrogenation of the product; or

b) by reaction of a tertiary amide of an acid of formula 17

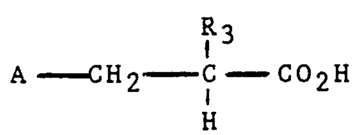

(17)

wherein $R_3$, $R_4$, $R_8$, $R_9$ and $R_{10}$ have the meanings mentioned in Claim 1, with a compound of formula 11

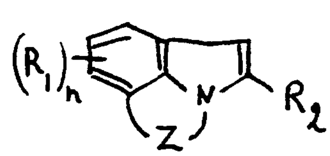

(11)

wherein $R_1$, $R_2$, n and Z have the meanings mentioned in Claim 1, or

c) by metalation of a compound of formula 2, wherein $R_9$ is a hydrogen atom and $R_1$, $R_2$, $R_3$, $R_4$, $R_8$, $R_{10}$, n and Z have the meanings mentioned in Claim 1, and then converting the metal compound with a compound of the formula $R_9$-X, wherein $R_9$ is alkyl having 1-6 C-atoms and X has the above-mentioned

meaning, or

d) by reaction of a compound of formula 18

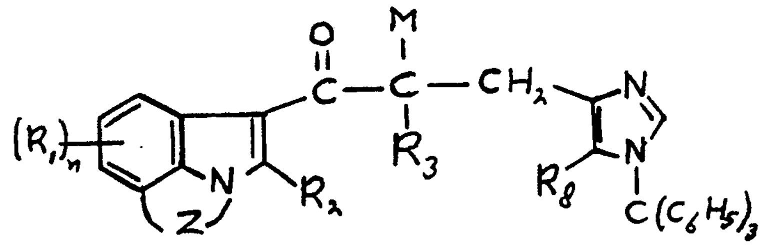

(18)

with a compound of the formula $R_4$-X, in which formulae $R_1$, $R_2$, $R_3$, $R_8$, n and Z have the meanings mentioned in Claim 1, and X is a group which may be replaced by a nucleophile, and then splitting off the trityl group.

European Patent Office

# PARTIAL EUROPEAN SEARCH REPORT

which under Rule 45 of the European Patent Convention shall be considered, for the purposes of subsequent proceedings, as the European search report

Application number
EP 89 20 3204

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 242 973 (GLAXO GROUP LTD.)<br>* Claims * | 1-3,<br>5,6 | C 07 D 471/06<br>C 07 D 513/06<br>A 61 K 31/435;<br>A 61 K 31/54<br>A 61 K 31/55<br>C 07 D 487/06//<br>(C 07 D 471/06,<br>C 07 D 221:00,<br>C 07 D 209:00)<br>(C 07 D 487/06,<br>C 07 D 223:00,<br>C 07 D 209:00)<br>(C 07 D 513/06,<br>C 07 D 279:00,<br>C 07 D 209:00) |
| | -- | | |
| A | EP-A-0 139 584 (SYNTHELABO)<br>* Page 15; claims * | 1-3 | |
| | ---- | | |

TECHNICAL FIELDS SEARCHED (Int. Cl.4)

C 07 D 471/00
C 07 D 487/00
C 07 D 513/00
A 61 K 31/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-3,5-6

Claims searched incompletely:

Claims not searched: 4

Reason for the limitation of the search:

Method for treatment of the human or animal body by surgery or therapy (see Art. 52(4) of the European Patent Convention).

| Place of search | Date of completion of the search | Examiner |
|---|---|---|
| THE HAGUE | 02-04-1990 | DE JONG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82